# EUROPEAN PATENT SPECIFICATION

(11) **EP 2 012 722 B1**
(45) Date of publication and mention of the grant of the patent: **13.04.2011**
(21) Application number: 07761274.5
(22) Date of filing: 25.04.2007
(51) Int. Cl.: A61F 9/00

(54) **MULTI-PURPOSE PHACOEMULSIFICATION NEEDLE**
MEHRZWECK-PHAKOEMULSIFIKATIONSNADEL
AIGUILLE DE PHACOÉMULSIFICATION POLYVALENTE

(30) Priority: 02.05.2006 US 416950
(43) Date of publication of application: 14.01.2009
(73) Proprietor: Abbott Medical Optics Inc., Santa Ana, CA 92705-4933 (US)
(72) Inventor: KADZIAUSKAS, Kenneth, E., Coto De Caza, CA 92679 (US); STEEN, Mark, Santa Ana, CA 92705 (US); SUTTON, Thomas, B., Orange, CA 92867 (US)
(74) Representative: HOFFMANN EITLE
(86) International application number: PCT/US2007/067410
(87) International publication number: WO 2007/130829

(56) References cited:
- WO-A-01/74427
- WO-A-94/22402
- US-A1- 2004 193 121

## Description

### Related Application

This application is a continuation-in-part of U.S. Serial No. 10/817,684 filed April 2, 2004, now U.S. Patent No. 7,037,296, issued May 2, 2006, which is a continuation-in-part of U.S. Serial No. 10/115,626 filed April 4, 2002, now U.S. Patent No. 6,958,056, issued October 25, 2005.

### Background of the Invention

### Field of the Invention

The present invention generally relates to phacoemulsification needles and is more particularly directed to a multipurpose phacoemulsification needle.

Phacoemulsification refers to a method of lens and cataract extraction from an eye. The procedure includes an ultrasonically vibrated needle which is inserted through a very small incision of the cornea in order to provide energy for fragmenting the lens and cataract which then can be aspirated and removed through the incision.

The needle is supported by a handpiece interconnected with a console which provides electrical power to the handpiece as well as a supply of irrigation fluid and a vacuum source for aspiration of fragmented tissue and liquids.

The handpiece typically includes piezoelectric crystals or magnetostuctive elements which are coupled to the needle.

Often several needle types are utilized in the phacoemulsification procedure. For example, many needles utilize a relatively sharp surface in order to both enhance phacoemulsification and to break up the lens nucleus and cortex. However, any sharp edges can inadvertently cut the capsule surrounding the lens which may impair effective healing and prevent satisfactory visual recovery.

It is necessary, however, to remove soft cortical remnants of cataract tissue against the capsule and this often necessitates a second needle, or tool. This process effectively vacuums the internal surface of the lens capsule and is known as polishing.

The present invention provides for a multipurpose phacoemulsification needle suitable for both phacoemulsification of cataract and lens tissue as well as being affective for polishing the capsule.

### Summary of the Invention

A multipurpose phacoemulsification needle in accordance with the present invention includes the technical features of independent claim 1.

The needle body includes a proximal end adapted for attachment to a phacoemulsification handpiece and a distal end having a tip portion.

Preferably, the portion includes a truncated hemisphere having a flat surface thereon and a port disposed in the flat surface with the port communicating with the needle body lumen.

The tip can also be described as having a convex surface of revolution about a centerline of the tip portion which is defined by an arc extending from a circumference of the needle body to the tip portion centerline.

The surface of revolution preferably provides for a rounded portion which includes sufficient area for polishing the eye lens capsule.

More particularly, the flat surface may extend from a centerline of the top portion to a tip portion circumference and include a bevel in the flat surface surrounding the port. This features insures that there will be no or minimal sharp edges in the needle distal end.

Still more particularly, the needle flat surface may be disposed at about a 45° angle with respect to the tip potion centerline.

The needle body may include a curved portion disposed adjacent the distal end tip portion which includes a curvature in a plane not including the flat surface.

Preferably, the plane is perpendicular to the flat surface and in one embodiment, the flat surface faces inward from an arc established by the needle body curved portion and in another embodiment, the flat surface faces outwardly from an arc established by the needle body curved portion.

The curved needle embodiments have additional advantage in the generation of cavitational energy, manipulating tissue within the eye during surgery and accessing cortex material from difficult to access locations within the eye during irrigation and aspiration.

The multi-purpose phacoemulsification needle comprises a needle body disposed about a centerline and having a coaxial lumen therethrough. The needle further comprises a distal tip comprising a flat surface with a port disposed therein. The flat surface is disposed at an acute angle relative to a portion of the centerline intersected by a line passing through the flat surface. The distal tip includes a continuous surface extending from a distal portion of said coaxial lumen to a distal portion of the needle body and free of
The US 2004/0193121 A1 discloses a multipurpose phacoemulsification needle according to the pre-characterizing portion of claim 1. Similar needles which are helpful as background information are disclosed in WO 94/22402 and WO 01/74427 A1. discontinuities able to initiate a tear in a lens capsule of an eye when the capsule is invaginated by the phacoemulsification needle under normal ocular aspiration conditions.

### Brief Description of the Drawings

The advantage of the present invention may be more readily understood by consideration of the following detailed description, particularly in conjunction with the accompanying drawings in which:
Figure 1 is perspective view of a multipurpose phacoemulsification needle generally showing a needle body having a proximal and distal end with a tip portion disposed at the end;
Figure 2 is a cross-sectional view of the tip portion of the needle shown in Figure 1;
Figure 3 is a representation of the needle function in polishing an eye capsule;
Figure 4 is a perspective view of an alternative embodiment similar to the embodiment shown in Figure 1 but with a curved needle body portion;
Figure 5 is a perspective view of yet another embodiment similar to the embodiment shown in Figure 4 with a curved needle body portion, the curve being opposite that shown in Figure 4; and
Figure 6 is a cross-sectional view of the tip portion of the needle of the present invention shown in Figure 1.

### Detailed Description of the Drawings

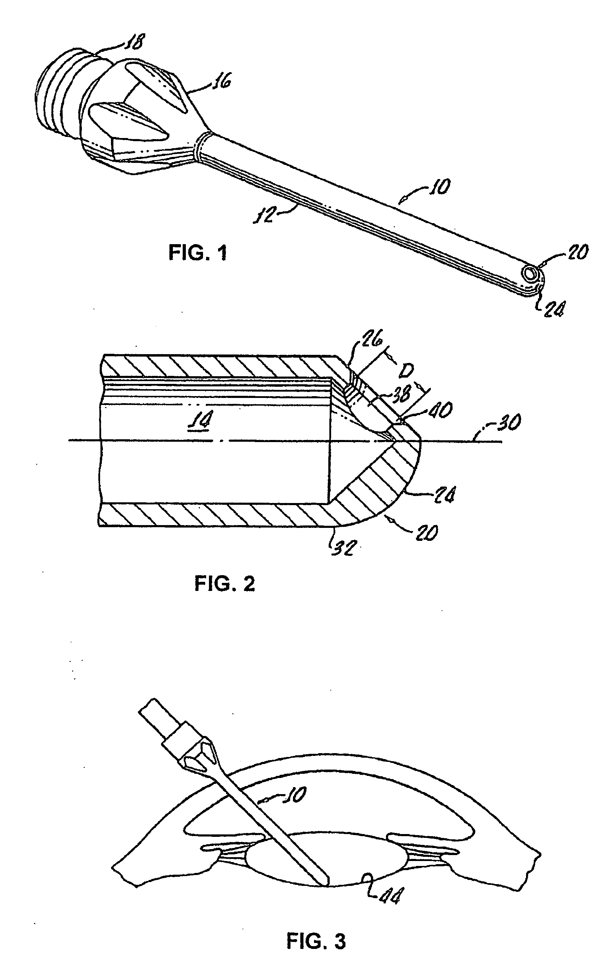

With reference to Figure 1, there is shown a multipurpose phacoemulsification needle 10 generally having a needle body 12 having a lumen 14 therethrough as shown in Figure 2. The needle body includes a proximal end 16 which may include a threaded portion 18 which adapts the needle for attachment to a phacoemulsification handpiece, not shown. The needle 10 may be formed from a single piece of material suitable for phacoemulsification needle as is well known in the art.

A tip portion 20 of the needle body 12 includes a truncated hemisphere 24 having a flat surface 26 thereon. The flat surface 26 is preferably disposed from a location at or near the centerline 30 to a location at a distal end of the needle body 12.

The hemispherical surface 24 is formed by a convex surface of revaluation about a centerline 30 of the tip portion 20 defined by an arc extending from a circumference 32 of the needle 12 to the centerline 30.

The hemispherical or rounded surface 24 is of sufficient area for polishing an eye lens capsule as will be hereinafter described.

A port 38 is disposed in a flat surface 26 with the port 38 communicating with the needle body lumen 14 as most clearly shown in Figure 2. A bevel 40 in the flat surface 26 surrounding the port, provides for a smooth entry through the port 38 and eliminates any sharp edges.

Preferably, the flat surface 26 is disposed at an angle of about 45degrees (e.g., to within typical engineering tolerance, for example, 45 degrees ±2 degrees or 45 degrees ± 1 degree) with a centerline 30. In some embodiments, the flat surface 26 is disposed at an angle of between about 25 degrees and about 65 degrees, preferably between 35 degrees and 55 degrees. In certain embodiments, the port diameter, D, is between 0.1 mm to about 0.5 mm, preferably between 0.15 mm and 0.45 mm, more preferably 0.2 mm to 0.4 mm or about 0.3 mm. In certain embodiments, the diameter, D, is chosen so as to restrict the aspiration flow rate of the needle 10 to be at or below a predetermined value, for example, so as to prevent the aspiration flow rate from exceeding a predetermined flow rate when an occlusion of the aspiration line breaks loose or is cleared.

Because of the rounded surface 24, and position of the port at a 45 degrees angle, the needle 10 is well suited for either cataract extraction and/or Irrigation and Aspiration (I/A) of the cortex. For example, the angled flat surface 26 may be disposed upon the needle 10 so that it may be conveniently directed toward the natural lens of an eye when phacoemulsification power is being used to remove portions of the natural lens. Later, when an I/A procedure is performed during the same surgical procedure, the rounded surface 24 of the same handpiece and needle 10 may be used to polish the lens capsule.

As illustrated in Figure 3, the curved nature of the tip portion 20 and the significant area of the rounded portion 24, enables the surgeon to work close to a capsule and, in fact polish the capsule 44. Through the use of ultrasonic energy the needle may also be used (during I/A irrigation aspiration function) to remove cortex. Thus, a specific and important advantage of the present invention is that it eliminates the current need to use a separate handpiece to perform the I/A of a phacoemulsification procedure.

The present invention is easily distinguished over heretofore available phacoemulsification, such as for example, set forth in U.S. 5,980,529 which illustrates an off axis entry port but utilizes a angular or pointed end which is not amendable for lens capsule 44 polishing, and accordingly, is not a multipurpose needle.

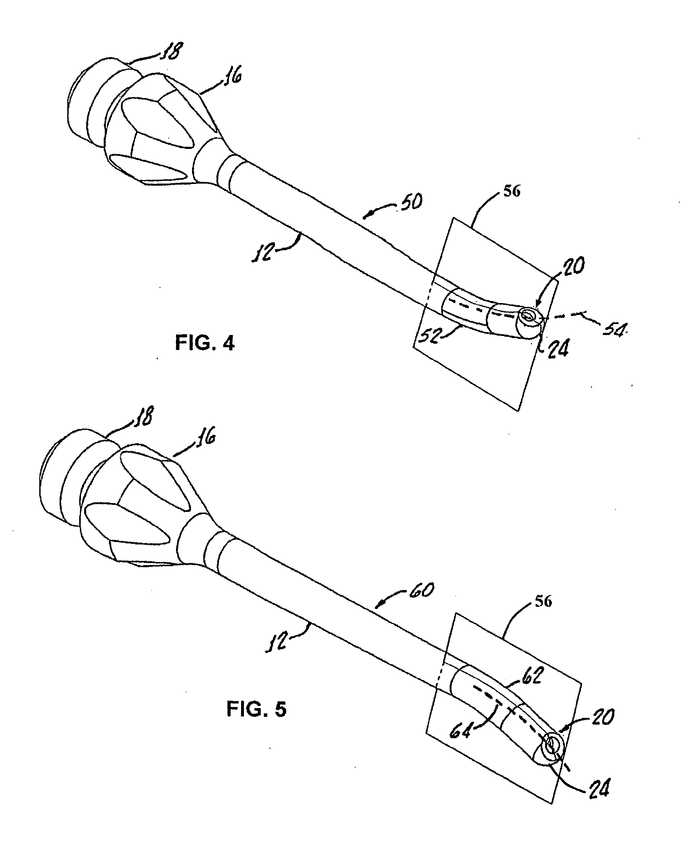

With reference to Figure 4, there is shown an alternative embodiment 50 accordance with the present invention with common reference characters indicating substantially similar or identical elements of the invention as hereinabove described in connection with the embodiment shown in Figure 1.

The multipurpose phacoemulsification needle 50 includes a curved portion 52 adjacent to the tip portion 20 having a curvature 54 in a plane 56 not including the flat surface 26, see Figure 2. Preferably, the flat surface 26 is perpendicular to the plane 56 established by the curved portion curvature, or arc, 54.

In the embodiment 50, a flat surface 26 faces inwardly from the arc 54 established by the needle body curved portion 52.

With reference to Figure 5, there is shown yet another embodiment 60 of a multipurpose phacoemulsification needle in accordance with the present invention with common reference numbers representing identical or substantially similar elements as hereinabove discussed in connection with Figures 1 and 4.

The phacoemulsification needle 60 is similar to the embodiment 50 shown in Figure 4 except that a curved portion 62 is provided proximate the tip 20 which establishes an arc 64 opposite the arc 54 shown in Figure 4 and in which the flat surface 64 faces outwardly from the arc 64 established by the needle body curved portion 62.

As hereinabove noted, these various phacoemulsification needle embodiments have the additional advantage in the generation of cavitational energy, manipulating tissue within the eye during surgery, as hereinabove noted, and accessing cortex material from difficult to access locations within the eye during irrigation and aspiration. The angle of curvature, or arc, 54, 64 may vary in the angle of the curvature or arc, the curvature depending upon the specific use intended for the needle 50, 60.

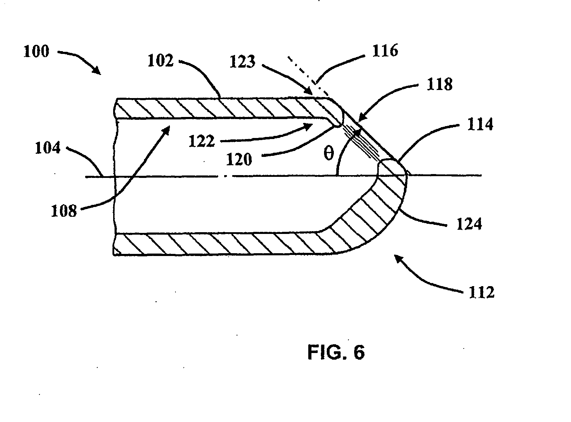

FIG. 6. illustrates a multi-purpose phacoemulsification needle 100 having a needle body 102. The needle body 102 may, for example, be straight, like the needle body illustrated in FIG. 1, curved like the needle body illustrated in FIG. 4, or some other shape suitable for an ocular surgical procedure. The needle body 102 is disposed about a centerline 104 and comprises a lumen 108 therein. The centerline 104 may be straight, as shown in FIG. 6, or may have at least a distal portion that is curved (e.g., like the arcs 54, 64 shown in FIGS. 4 and 5, respectively). The needle 100 further comprises a proximal end (not shown) and a distal tip 112. The proximal end is configured for attachment to a phacoemulsification handpiece. The distal tip 112 comprises a flat surface 114 disposed at an acute angle θ relative to a portion of the centerline 104 intersected by a line 116 passing through the flat surface 114. The distal tip 112 further comprises an opening or port 118 disposed within the flat surface 114 and communicating with the needle body lumen 108. The distal tip 112 includes a continuous surface 120 that is free of discontinuities and extending from a distal portion 122 of the lumen 108 to a distal portion 123 of the needle body 102. Preferably, the continuous surface 120 smoothly blends into a rounded surface 124 generally disposed and having sufficient area for polishing the lens capsule of an eye.

As used herein, the term discontinuity refers to a transition between two surfaces or surface portions that produce a visible corner or edge. While all physical corners and edges ultimately have a radius, a discontinuity, as it is used herein, pertains to a corner or edge that is visible as a discrete feature. As used in this context, "visible" refers to visible as seen by the naked eye, or with the assistance of certain low-power magnification devices, such as an ocular or a loupe. Another way of defining a discontinuity, as used herein, is as a portion or feature of surface at the distal tip of a phacoemulsification needle that has a sharp edge or a radius sufficiently small to initiate or propagate a tear in the wall of a lens capsule of a human eye when the capsule is invaginated by the needle under typical ocular surgical aspiration conditions.

The angle θ of the flat surface 114, combined with the inclusion of the rounded surface 124, allow the multi-purpose phacoemulsification needle 100 to be used advantageously in both phacoemulsification procedures and I/A procedures in which phacoemulsification power either is not supplied to the needle 100, or is supplied only for short periods of time, for example, on an as-needed bases to break apart lens material that causes the aspiration flow to become temporarily blocked or occluded. Preferably, the flat surface 114 is disposed at an angle of about 45° degrees (e.g., to within typical engineering tolerance). In some embodiments, the flat surface 26 is disposed at an angle of between about 25 degrees and 65 degrees, preferably between 35 degrees and 55 degrees. In other embodiments, the flat surface 114 is replaced by or combined with a concave or some other arcuate surface that is configured to help focus phacoemulsification power to a predetermined location in front of and/or above the needle 100. The single flat surface 114 may also be replaced by two or more surface portions that are either flat or arcuate and that are disposed at different angles relative to the centerline 104.

The continuous surface 120 enhances the versatility of the multi-purpose phacoemulsification needle 100 by, for example, allowing the surgeon to advantageously perform a variety of techniques with a single handpiece. For example, during some surgical procedures, instead of using the rounded surface 124, a surgeon may prefer to break apart or emulsify relatively soft and/or sticky cortical material remaining on the capsule surface by invaginating the capsule surface inside the needle 100. As with the needle 100, inner and outer surfaces of the distal tip 212 of phacoemulsification needle 200 are preferably configured to be smooth and free of any sharp edges or corners in order to protect the capsule surface from tearing during invagination.

Although there has been hereinabove described a specific curved multi-purpose phacoemulsification needle in accordance with the present invention for the purpose of illustrating the manner in which the invention may be used to advantage, it should be appreciated that the invention is not limited thereto. That is, the present invention may suitably comprise, consist of, or consist essentially of the recited elements. Further, the invention illustratively disclosed herein suitably may be practiced in the absence of any element which is not specifically disclosed herein. Accordingly, any and all modifications, variations or equivalent arrangements which may occur to those skilled in the art, should be considered to be within the scope of the present invention as defined in the appended claims.

## Claims

1. A multi-purpose phacoemulsification needle, comprising:
a needle body (**102**) disposed about a centerline (104) and having a coaxial lumen (**108**) therethrough;
a proximal end adapted for attachment to a phacoemulsification handpiece; and
a distal tip (112) comprising:
a flat surface (114) disposed at an acute angle (θ) relative to a portion of the centreline intersected by a line (**116**) passing through the flat surface; **and**
a port (118) disposed within the flat surface and communicating with the needle body lumen;
**characterized in that**
the **distal tip** (**112**) **of the** multi-purpose phacoemulsification needle further comprises a continuous surface (120) extending from a distal portion (122) of said coaxial lumen (108) to a distal portion (123) of **an outer surface of** the needle body (102) and free of discontinuities able to initiate a tear in a lens capsule of an eye when the capsule is invaginated by the phacoemulsification needle under normal ocular aspiration conditions.

2. The multi-purpose phacoemulsification needle of claim 1, wherein the angle (θ) is about 45 degrees.

3. The multi-purpose phacoemulsification needle of claim 1, wherein the angle (θ) is between about 25 degrees and 65 degrees.

4. The multi-purpose phacoemulsification needle of claim 1, wherein the port (**118**) has a diameter that is less than about 0.5 mm.

5. The multi-purpose phacoemulsification needle of claim 1, wherein the port (**118**) has a diameter that is between about 0.2 mm and about 0.4 mm.

6. A multi-purpose phacoemulsification needle according to any one of claims 1 to 5, wherein the distal tip (**112**) comprises:
a rounded bottom portion having a surface area configured for polishing **the** eye lens capsule; and
a top portion comprising the flat surface (**114**) disposed from a location at or near the centerline (**104**) to a location at a distal end of the needle body (**102**).

## Patentansprüche

1. Eine Mehrzweck-Phakoemulsifikationsnadel, umfassend:
einen Nadelkörper (102), angeordnet um eine Mittellinie (104) und ein durchgehendes koaxiales Lumen (108) aufweisend;
ein proximales Ende, angepasst zum Anbringen an ein Phakoemulsifikationshandstück; und eine distale Spitze (112), umfassend:
eine ebene Oberfläche (114), angeordnet in einem spitzen Winkel (θ) relativ zu einem Abschnitt der Mittellinie, geschnitten durch eine Linie (116), durch die ebene Oberfläche durchtretend; und
eine Mündung (118), angeordnet innerhalb der ebenen Oberfläche und kommunizierend mit dem Nadelkörperlumen;
**dadurch gekennzeichnet, dass**
die distale Spitze (112) der Mehrzweck-Phakoemulsifikationsnadel des Weiteren eine kontinuierliche Oberfläche (120) umfasst, sich erstreckend von einem distalen Abschnitt (122) des koaxialen Lumens (108) zu einem distalen Abschnitt (123) einer äußeren Oberfläche des Nadelkörpers (102) und frei von Unstetigkeiten in der Lage, einen Riss in einer Linsenkapsel eines Auges zu initiieren, wenn die Kapsel durch die Mehrzweck-Phakoemulsifikationsnadel unter normalen okkularen Aspirationsbedingungen eingestülpt ist.

2. Die Mehrzweck-Phakoemulsifikationsnadel gemäß Anspruch 1, wobei der Winkel (θ) ungefähr 45 Grad beträgt.

3. Die Mehrzweck-Phakoemulsifikationsnadel gemäß Anspruch 1, wobei der Winkel (θ) ungefähr zwischen 25 Grad und 65 Grad beträgt.

4. Die Mehrzweck-Phakoemulsifikationsnadel gemäß Anspruch 1, wobei die Mündung (118) einen Durchmesser aufweist, der kleiner als ungefähr 0,5 mm ist.

5. Die Mehrzweck-Phakoemulsifikationsnadel gemäß Anspruch 1, wobei die Mündung (118) einen Durchmesser aufweist, der zwischen ungefähr 0,2 mm und ungefähr 0,4 mm beträgt.

6. Eine Mehrzweck-Phakoemulsifikationsnadel gemäß einem der Ansprüche 1 bis 5, wobei die distale Spitze (112) umfasst:
einen abgerundeten unteren Abschnitt, aufweisend eine Oberfläche, ausgelegt zum Polieren der Augenlinsenkapsel; und
einen oberen Abschnitt, umfassend die ebene Oberfläche (114), angeordnet ausgehend von einer Stelle an oder nahe der Mittellinie (104) zu einer Stelle an einem distalen Ende des Nadelkörpers (102).

## Revendications

1. Aiguille de phacoémulsification polyvalente, comprenant :
un corps d'aiguille (102) disposé autour d'un axe (104) et ayant une lumière coaxiale (108) à travers celui-ci ;
une extrémité proximale adaptée pour être fixée à une pièce à main de phacoémulsification ; et
une pointe distale (112) comprenant :
une surface plate (114) disposée au niveau d'un angle aigu (θ) par rapport à une partie de l'axe que coupe une ligne (116) passant à travers la surface plate ; et
un orifice (118) disposé dans la surface plate et communiquant avec la lumière du corps d'aiguille ;
**caractérisée en ce que**
la pointe distale (112) de l'aiguille de phacoémulsification polyvalente comprend en outre une surface continue (120) s'étendant de la partie distale (122) de ladite lumière coaxiale (108) à une partie distale (123) d'une surface extérieure du corps d'aiguille (102) et sans discontinuités capable de déclencher une déchirure dans une capsule du cristallin d'un oeil lorsque la capsule est invaginée par l'aiguille de phacoémulsification sous des conditions d'aspiration oculaire normales.

2. Aiguille de phacoémulsification polyvalente de la revendication 1, dans laquelle l'angle (θ) est d'environ 45 degrés.

3. Aiguille de phacoémulsification polyvalente de la revendication 1, dans laquelle l'angle (θ) est entre environ 25 degrés et 65 degrés.

4. Aiguille de phacoémulsification polyvalente de la revendication 1, dans laquelle l'orifice (118) a un diamètre qui est inférieur à environ 0,5 mm.

5. Aiguille de phacoémulsification polyvalente de la revendication 1, dans laquelle l'orifice (118) a un diamètre qui est entre environ 0,2 mm et environ 0,4 mm.

6. Aiguille de phacoémulsification polyvalente selon l'une quelconque des revendications 1 à 5, dans laquelle la pointe distale (112) comprend :
une surface inférieure arrondie ayant une surface configurée pour polir la capsule du cristallin de l'oeil ; et
une surface supérieure comprenant la surface plate (114) disposée d'un emplacement au niveau ou à proximité de l'axe (104) à un emplacement au niveau d'une extrémité distale du corps d'aiguille (102).
